# EUROPEAN PATENT APPLICATION

(11) **EP 4 806 983 A1**
(43) Date of publication of application: **16.09.2026**
(21) Application number: 24888627.7
(22) Date of filing: 31.10.2024
(51) Int. Cl.: C12M 1/42

(54) **ULTRASONIC WAVE IRRADIATION APPARATUS**

(30) Priority: 10.11.2023 JP 2023192558
(71) Applicant: The University of Osaka, Suita-shi, Osaka 565-0871 (JP); Daicel Corporation, Osaka-shi, Osaka 530-0011 (JP)
(72) Inventor: OGI, Hirotsugu, Suita-shi, Osaka 565-0871 (JP); NAKAJIMA, Kichitaro, Suita-shi, Osaka 565-0871 (JP); GOTO, Yuji, Suita-shi, Osaka 565-0871 (JP); YAMAGUCHI, Keiichi, Suita-shi, Osaka 565-0871 (JP); MATSUDA, Hirokazu, Tokyo 108-8230 (JP); NAGAMATSU, Shinji, Tokyo 108-8230 (JP)
(74) Representative: Müller-Boré & Partner Patentanwälte PartG mbB
(86) International application number: PCT/JP2024/038842
(87) International publication number: WO 2025/100330

(57) **Abstract**

An ultrasonic wave irradiation apparatus includes an ultrasonic wave generation device including a vibration element configured to generate ultrasonic waves by vibrating, wherein the ultrasonic wave generation device includes a container installation part in which an irradiation target container that accommodates an object to be irradiated with the ultrasonic waves is to be installed, and propagates the ultrasonic waves generated by the vibration element to the irradiation target container through a solid

## Description

### Technical Field

The present invention relates to an ultrasonic wave irradiation apparatus.

### Background Art

The usefulness of ultrasonic waves has been confirmed in various fields. Ultrasonic waves are used for, for example, pulverization, suspension, micronization, dissolution, stirring, heating, aggregation, acceleration of reactions, and the like. It is also known that ultrasonic waves are used to promote the formation of amyloid fibrils and to fragment and micronize the amyloid fibrils. In recent years, a technique of applying characteristics of ultrasonic waves has been attracting attention, and an ultrasonic wave irradiation apparatus capable of irradiating an object with ultrasonic waves has been developed.

In this connection, an ultrasonic wave irradiation apparatus is known that generates amyloid by irradiating a supersaturated solution of an amyloid-causing protein contained in a well with ultrasonic waves (for example, Patent Document 1). The ultrasonic wave irradiation apparatus disclosed in Patent Document 1 includes a treatment tank (container) in which a medium (water) composed of a fluid is accommodated, an ultrasonic wave generation device provided outside the treatment tank, and a sample plate having a plurality of wells immersed in the medium in the treatment tank, and irradiates a solution in each well with ultrasonic waves by propagating the ultrasonic waves generated by the ultrasonic wave generation device through the medium.

### Citation List

### Patent Document

Patent Document 1: JP 2023-060987 A

### Summary of the Invention

### Problems to be solved by the invention

Since an ultrasonic wave irradiation apparatus in the related art irradiates an object with ultrasonic waves by propagating the ultrasonic waves through a fluid, a container is necessary to store the fluid, resulting in an increase in the size of the device. In addition, there are problems in that energy efficiency is low because the attenuation of the ultrasonic waves is large in fluid propagation compared with solid propagation and the structure of the entire apparatus is complicated because a fluid degassing device for suppressing the generation of air bubbles is essential.

The technique according to the present disclosure has been made to solve such problems, and an object thereof is to implement miniaturization of an ultrasonic wave irradiation apparatus, simplification of a structure, and improvement of energy efficiency.

### Means for solving the Problems

To solve the above problems, an ultrasonic wave irradiation apparatus according to the present disclosure adopts the following configuration. That is, the gist of the technique according to the present disclosure is described below.
[1] An ultrasonic wave irradiation apparatus includes an ultrasonic wave generation device including a vibration element configured to generate ultrasonic waves by vibrating,
   wherein the ultrasonic wave generation device includes a container installation part in which an irradiation target container configured to accommodate an object to be irradiated with the ultrasonic waves is to be installed, and propagates the ultrasonic waves generated by the vibration element to the irradiation target container through a solid.
[2] The ultrasonic wave irradiation apparatus according to [1], in which the ultrasonic wave generation device further includes a first propagation part configured to propagate the ultrasonic waves through a solid, and
   the first propagation part includes the container installation part and propagates the ultrasonic waves to the irradiation target container.
[3] The ultrasonic wave irradiation apparatus according to [1] or [2], in which the container installation part is provided with a holding portion holding an acoustic coupling agent between the ultrasonic wave generation device and the irradiation target container, the acoustic coupling agent being configured to propagate the ultrasonic waves.
[4] The ultrasonic wave irradiation apparatus according to any one of [1] to [3], further including a rotation mechanism configured to rotate the irradiation target container installed in the container installation part relative to the ultrasonic wave generation device about a rotation axis arranged along an arrangement direction of the vibration element and the container installation part.
[5] The ultrasonic wave irradiation apparatus according to [4], in which the rotation mechanism includes:
   a shaft receiving hole penetrating the ultrasonic wave generation device along the rotation axis;
   a shaft portion inserted through the shaft receiving hole and connected to the irradiation target container in a manner that the shaft portion is rotatable integrally with the irradiation target container; and
   a rotation device disposed on an opposite side of the container installation part with respect to the vibration element part in the arrangement direction, the rotation device being connected to the shaft portion and configured to rotate the shaft portion about the rotation axis.
[6] The ultrasonic wave irradiation apparatus according to [4] or [5], in which the irradiation target container includes one or more containing parts arrayed in a circumferential direction about the rotation axis and configured to accommodate the object,
   the container installation part is provided with a holding portion holding an acoustic coupling agent between the ultrasonic wave generation device and the one or more containing parts, the acoustic coupling agent being configured to propagate the ultrasonic waves, and
   the holding portion is formed as an annular groove extending in the circumferential direction about the rotation axis.
[7] The ultrasonic wave irradiation apparatus according to any one of [1] to [6], in which the container installation part is formed as a recessed part configured to accommodate the irradiation target container and provided with an open end on one side, and
   the ultrasonic wave irradiation apparatus further includes a lid member enabling opening and closing of the open end of the recessed part.
[8] The ultrasonic wave irradiation apparatus according to [7], in which a window portion is formed in at least one of the container installation part and the lid member, the window portion allowing fluorescence measurement of the object to be performed from an outside of the recessed part.
[9] The ultrasonic wave irradiation apparatus according to any one of [1] to [8], further including a temperature control device configured to adjust a temperature of the object.
[10] The ultrasonic wave irradiation apparatus according to [2], in which the ultrasonic wave generation device includes a second propagation part disposed on an opposite side of the first propagation part with the vibration element interposed between the second propagation part and the first propagation part, the second propagation part being configured to propagate the ultrasonic waves generated by the vibration element through a solid.
[11] The ultrasonic wave irradiation apparatus according to [1], in which the container installation part is formed over the vibration element.
[12] The ultrasonic wave irradiation apparatus according to [11], in which the irradiation target container includes one or more containing parts circumferentially arrayed and configured to accommodate the object, and
   the vibration element extends annularly along the one or more wells.
[13] The ultrasonic wave irradiation apparatus according to any one of [1] to [12], further including the irradiation target container.

### Effects of the invention

According to the present disclosure, an ultrasonic wave irradiation apparatus can be decreased in size.

### Brief Description of the Drawings

FIG. 1 is a longitudinal cross-sectional view of an ultrasonic wave irradiation apparatus according to an embodiment.
FIG. 2 is a perspective view of a sample plate according to the embodiment.
FIG. 3 is a perspective view of an ultrasonic wave generation device according to the embodiment.
FIG. 4 is a diagram illustrating an example of a mode of connection between a shaft portion of a rotation mechanism and the sample plate.
FIG. 5 is a longitudinal cross-sectional view of an ultrasonic wave irradiation apparatus according to a first variation of the embodiment.
FIG. 6 is a longitudinal cross-sectional view of an ultrasonic wave irradiation apparatus according to a second variation of the embodiment.

### Mode for Carrying Out the Invention

Hereinafter, embodiments of the present disclosure are described. Note that each of the configurations, combinations thereof, and the like in the embodiments is an example, and various additions, omissions, substitutions, and other changes of the configurations may be made as appropriate without departing from the spirit of the present disclosure. The present disclosure is not limited by the embodiments and is limited only by the claims. Further, a numerical range expressed by "to" implies a range including the numerical values described before and after "to" as the lower limit value and the upper limit value. Specifically, "A to B" implies a value that is A or more and B or less.

### Overall Configuration

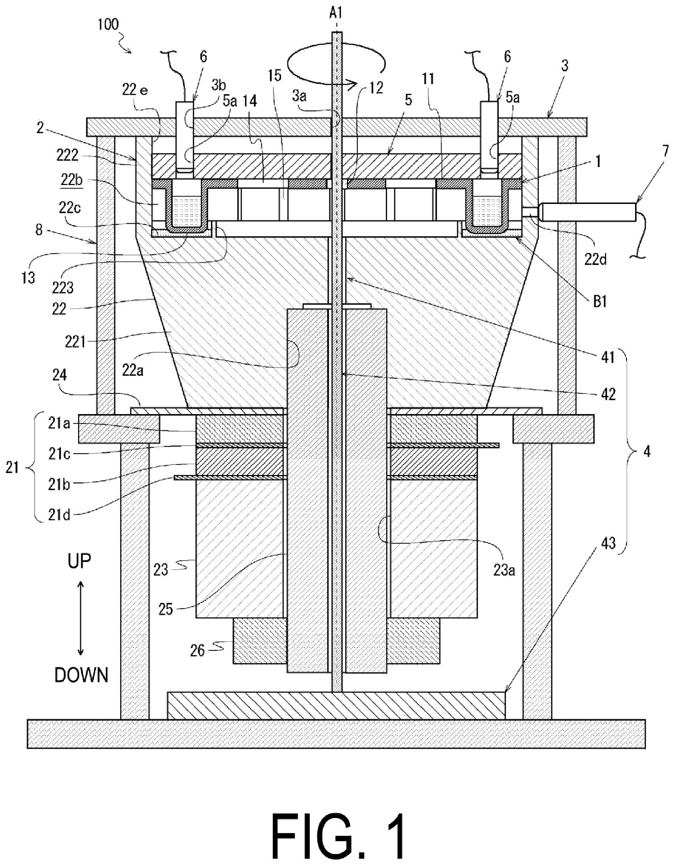

FIG. 1 is a longitudinal cross-sectional view of an ultrasonic wave irradiation apparatus 100 according to an embodiment. FIG. 1 illustrates a cross section (referred to as a longitudinal cross section) along a rotation axis indicated by reference sign A1. In FIG. 1, an up-down direction of the ultrasonic wave irradiation apparatus 100 is indicated. In the present embodiment, the up-down direction is defined as the arrangement direction of piezoelectric elements indicated by reference signs 21a and 21b and a container installation part indicated by reference sign 22b. In the arrangement direction of the piezoelectric elements 21a and 21b (vibration elements) and the container installation part 22b, the side of the piezoelectric elements 21a and 21b is a lower side, and the side of the container installation part 22b is an upper side. Although two piezoelectric elements are used in the present example, the number of piezoelectric elements may be 1. The number of piezoelectric elements is not particularly limited, and is usually 1 to 4, preferably 1, 2, or 4, more preferably 1 or 2, particularly preferably 2. The rotation axis A1 is arranged along the up-down direction. The ultrasonic wave irradiation apparatus 100 is used in a state where the up-down direction of the ultrasonic wave irradiation apparatus 100 coincides with a vertical direction (direction of gravity) and an upper side (side of the container installation part 22b) of the ultrasonic wave irradiation apparatus 100 coincides with an upper side in the vertical direction. However, the technique of the present disclosure is not limited to such a configuration.

The ultrasonic wave irradiation apparatus 100 according to the present embodiment irradiates an object to be irradiated with ultrasonic waves (hereinafter, ultrasonic irradiation target) with ultrasonic waves, thereby causing a change such as pulverization, suspension, micronization, dissolution, stirring, heating, aggregation, or acceleration of reactions in the ultrasonic irradiation target. For example, the ultrasonic wave irradiation apparatus 100 uses, as the ultrasonic irradiation target, a solution (hereinafter, target solution L1) including proteins and fluorescent molecules that emit light when adsorbed to aggregates of the proteins. In the present embodiment, the proteins are, for example, amyloid β causing Alzheimer's disease, β2 microglobulin causing dialysis amyloidosis, or α-synuclein causing Parkinson's disease. When the proteins are amyloid β, β2 microglobulin, or α-synuclein, the fluorescent molecules are thioflavin T. Thioflavin T adsorbs to aggregates of amyloid β, β2 microglobulin, or α-synuclein and emits fluorescence. The ultrasonic wave irradiation apparatus 100 according to the present embodiment irradiates the target solution L1 with ultrasonic waves to generate aggregates of proteins by an aggregation reaction. The ultrasonic wave irradiation apparatus 100 irradiates the target solution L1 with excitation light and detects fluorescence emitted from the fluorescent molecules. However, the ultrasonic irradiation target according to the present disclosure is not limited to the above.

As illustrated in FIG. 1, the ultrasonic wave irradiation apparatus 100 includes a sample plate 1, an ultrasonic wave generation device 2, a lid member 3, a rotation mechanism 4, a temperature control device 5, a light irradiation device 6, a light detection device 7, and a housing 8. The components of the ultrasonic wave irradiation apparatus 100 are described below.

### Sample Plate

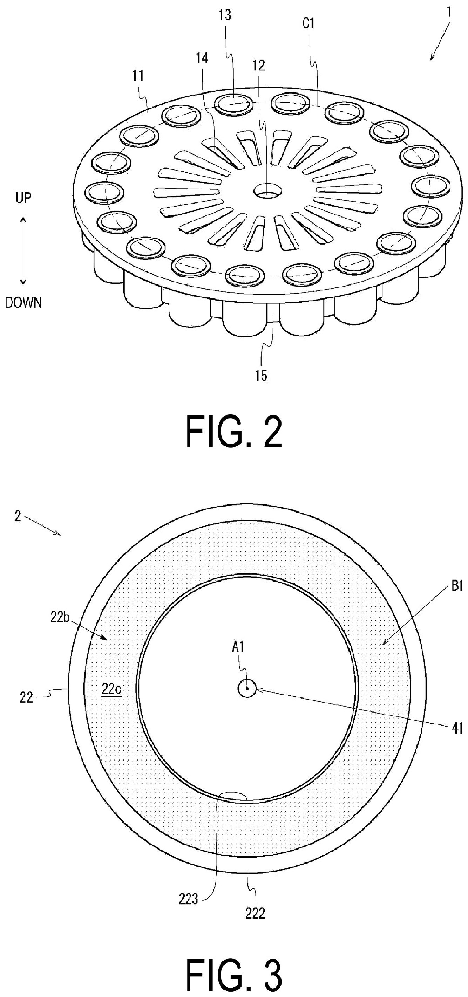

FIG. 2 is a perspective view of the sample plate 1. The sample plate 1 is a container in which the target solution L1 (ultrasonic irradiation target) is contained. The sample plate 1 is an example of an "irradiation target container" according to the present disclosure. The sample plate 1 includes a main body portion 11, a shaft receiving hole 12, a plurality of wells 13, a plurality of through holes 14, and a plurality of wall portions 15. The sample plate 1 is formed as a transparent body from a resin material such as polypropylene. The material of the irradiation target container according to the present disclosure is not particularly limited, and polypropylene having excellent ultrasonic wave permeability can be suitably used. In addition, as the material of the irradiation target container, an amorphous cyclic olefin resin having excellent ultrasonic wave and light transmittance may be used. The amorphous cyclic olefin resin is more preferable as the material of the irradiation target container from the viewpoint of excellent light transmittance.

The main body portion 11 is formed in a disk shape orthogonal to the up-down direction. The shaft receiving hole 12 is a through hole disposed in the center of the main body portion 11 and penetrating the main body portion 11 in the up-down direction. A shaft portion 42, which is described below, with the rotation axis A1 as a center axis is inserted through the shaft receiving hole 12.

The plurality of wells 13 constitute multiple channels of the sample plate 1. Each well 13 has a bottomed tubular shape extending in the up-down direction. An upper end portion of the well 13 is coupled to the main body portion 11 and opens in an upper surface of the main body portion 11. A lower end portion of the well 13 protrudes (hangs) downward from a lower surface of the main body portion 11 and is closed. The plurality of wells 13 are disposed at equal distances from the shaft receiving hole 12 and are arrayed in a circumferential direction about the shaft receiving hole 12 (that is, the rotation axis A1). More specifically, the plurality of wells 13 are arranged at predetermined intervals (equal intervals) along the circumference of a circle C1 centered on the shaft receiving hole 12. The sample plate 1 according to the present embodiment has 18 wells 13, for example. The plurality of wells 13 contain the target solution L1. The openings of the plurality of wells 13 are closed by a transparent sealing member (not illustrated). The plurality of wells 13 are an example of a plurality of containing parts according to the present disclosure. Note that in the present disclosure, the number of containing parts is not particularly limited and need not be more than one. The shape and the number of the containing parts are not particularly limited. For example, the well 13 may be turned upside down so that the openings closed by the transparent sealing member face downward (that is, toward the ultrasonic wave generation device), and the object may be irradiated with ultrasonic waves through the sealing member. In this case, the main body portion 11 is located at the center of each well 13 in the up-down direction.

The plurality of through holes 14 penetrate the main body portion 11 in the up-down direction and are disposed radially about the shaft receiving hole 12. More specifically, the plurality of through holes 14 are disposed between the shaft receiving hole 12 and the plurality of wells 13 and they are axially symmetric with respect to the shaft receiving hole 12.

The plurality of wall portions 15 are disposed between the wells 13 and 13 adjacent to each other in the circumferential direction, hang down from the lower surface of the main body portion 11, and couple the wells 13 and 13 to each other.

### Ultrasonic Wave Generation Device

As illustrated in FIG. 1, the ultrasonic wave generation device 2 includes a drive unit 21, a front mass 22 (an example of a "first propagation part" according to the present disclosure), a rear mass 23 (an example of a "second propagation part" according to the present disclosure), a flange 24, a bolt 25, and a nut 26. The ultrasonic wave generation device 2 is configured as a bolt-fastened Langevin type transducer in which the drive unit 21, the front mass 22, the rear mass 23, and the flange 24 are layered in the up-down direction and are integrated by being fastened by the bolt 25 and the nut 26. However, the ultrasonic wave generation device according to the present disclosure is not limited to the Langevin type transducer. In the ultrasonic wave generation device 2 according to the present embodiment, the front mass 22 and the rear mass 23 are disposed on opposite sides with the drive unit 21 interposed therebetween, and the flange 24 disposed at a node position of vibration is held by the housing 8. The resonance frequency of the ultrasonic wave generation device 2 is not particularly limited, and is set to, for example, 20 kHz to 300 kHz. The resonance frequency of the ultrasonic wave generation device 2 can be adjusted by changing the sizes, weights, materials, or the like of the front mass 22, the rear mass 23, and the like.

The drive unit 21 includes the piezoelectric elements 21a and 21b (an example of "vibration elements" according to the present disclosure) and electrode plates 21c and 21d, which are layered in the up-down direction. The piezoelectric elements 21a and 21b are piezoelectric ceramics (PZT piezoelectric elements) that have been subjected to polarization processing. An external oscillator (not illustrated) is electrically connected to the electrode plates 21c and 21d. The electrode plates 21c and 21d are terminals for applying a voltage of a predetermined frequency supplied from the oscillator to the piezoelectric elements 21a and 21b. The piezoelectric elements 21a and 21b generate ultrasonic waves by vibrating when a voltage is applied. The piezoelectric elements 21a and 21b and the electrode plates 21c and 21d are formed in an annular shape, and the bolt 25 is inserted therethrough. Although two piezoelectric elements are used in the present example, the number of piezoelectric elements is not particularly limited and may be one. In addition, the vibration element according to the present disclosure is not limited to the piezoelectric element. The vibration element according to the present disclosure may be a piezoelectric element (electrostrictive element) that excites (generates) ultrasonic waves by vibrating when a voltage is applied, or may be a magnetostrictive element that excites (generates) ultrasonic waves by vibrating with a vibrating magnetic field.

The front mass 22 and the rear mass 23 are metal block bodies that propagate ultrasonic waves generated by the piezoelectric elements 21a and 21b through a solid (propagate the ultrasonic waves through a solid as a medium). The front mass 22 and the rear mass 23 resonate with ultrasonic vibrations transmitted from the piezoelectric elements 21a and 21b, and expand and contract to vibrate in the up-down direction and a direction orthogonal to the up-down direction, thereby amplifying and propagating the ultrasonic vibrations. Examples of the material of the front mass 22 and the rear mass 23 include metal materials such as aluminum alloys, titanium, steel (S45C), and stainless steel, and among these materials, stainless steel resistant to rust (having corrosion resistance) is preferably used. However, the material of the first propagation part and the second propagation part according to the present disclosure is not limited to a metal material. The first propagation part and the second propagation part may be solid members that can propagate ultrasonic waves.

As illustrated in FIG. 1, the front mass 22 is disposed above the drive unit 21, and is fixed to the piezoelectric elements 21a and 21b with the flange 24 interposed therebetween. The front mass 22 includes a block body portion 221, a peripheral wall portion 222, and an upright wall portion 223.

The block body portion 221 is a member for propagating ultrasonic waves generated by the piezoelectric elements 21a and 21b through a solid, and is formed in a substantially truncated conical shape that increases in diameter upward. A center axis of the block body portion 221 coincides with the rotation axis A1. The block body portion 221 is formed with a non-through screw hole 22a that opens in a lower end surface of the block body portion 221 and extends along the up-down direction. A center axis of the screw hole 22a coincides with the rotation axis A1. The bolt 25 is inserted into the screw hole 22a, and a male screw portion formed on an outer periphery of the bolt 25 and a female screw portion formed on an inner periphery of the screw hole 22a are screwed together. In the present embodiment, the cross section of the block body portion 221 orthogonal to the rotation axis A1 is circular, and the shape is not limited thereto. For example, the cross section of the front mass 22 may be polygonal.

The peripheral wall portion 222 is formed in a tubular shape extending in the up-down direction, and extends upward from the peripheral edge of an upper end surface of the block body portion 221. A center axis of the peripheral wall portion 222 coincides with the rotation axis A1. The block body portion 221 and the peripheral wall portion 222 form the container installation part 22b, which is a recessed part in which the sample plate 1 can be installed, at an upper end portion of the front mass 22. More specifically, the container installation part 22b is a space surrounded by the upper end surface of the block body portion 221 and an inner peripheral surface of the peripheral wall portion 222. A height dimension (length in the up-down direction) of the peripheral wall portion 222 is larger than a height dimension of the sample plate 1. Therefore, the container installation part 22b is deeper than the height of the sample plate 1. The peripheral wall portion 222 is formed with a window portion 22d for enabling the light detection device 7 to detect fluorescence from the outside of the container installation part 22b. The window portion 22d is formed as a through hole penetrating the peripheral wall portion 222 in the radial direction. An opening at an upper end portion of the peripheral wall portion 222 forms an open end 22e of the container installation part 22b.

The upright wall portion 223 extends in the up-down direction, is formed in a tubular shape having a smaller diameter than the peripheral wall portion 222, and extends upward from the upper end surface of the block body portion 221. A center axis of the upright wall portion 223 coincides with the rotation axis A1. The height dimension of the upright wall portion 223 is smaller than the height dimension of the peripheral wall portion 222. The block body portion 221, the peripheral wall portion 222, and the upright wall portion 223 form, on the container installation part 22b, a holding portion 22c that can hold an acoustic coupling agent B1. The upright wall portion 223 prevents the acoustic coupling agent contained in the holding portion 22c from being ejected by ultrasonic waves and leaking to a shaft receiving hole 41 side.

FIG. 3 is a top view of the ultrasonic wave generation device 2 according to the embodiment. As illustrated in FIG. 3, the holding portion 22c is formed as an annular groove extending in the circumferential direction about the rotation axis A1. The holding portion 22c can hold the acoustic coupling agent B1 that can propagate ultrasonic waves. The front mass 22 is in contact with the sample plate 1 via the acoustic coupling agent B1. The acoustic coupling agent B1 need not be interposed between the front mass 22 and the sample plate 1, and the front mass 22 and the sample plate 1 may be in direct contact with each other.

The holding portion 22c can receive the plurality of wells 13 of the sample plate 1. The sample plate 1 is installed in the container installation part 22b in a state where bottom portions (lower end portions) of the plurality of wells 13 are received in the holding portion 22c.

As illustrated in FIG. 1, the acoustic coupling agent B1 is filled into the holding portion 22c, and thus is interposed between the front mass 22 of the ultrasonic wave generation device 2 and the well 13 of the sample plate 1. The acoustic coupling agent B1 may be any agent as long as it can remove air from between the front mass 22 and the well 13, and is preferably a gel agent with small attenuation of vibration. However, the form of the acoustic coupling agent according to the present disclosure is not particularly limited, and examples thereof include a gel form and a cream form. Water may be used as the acoustic coupling agent. When air is present between the ultrasonic wave generation device 2 and the sample plate 1 without use of the acoustic coupling agent B1, a so-called dry contact state is established, and ultrasonic waves propagate through space. By interposing the acoustic coupling agent B1 between the ultrasonic wave generation device 2 and the sample plate 1, attenuation of ultrasonic waves can be suppressed compared with air propagation, so that energy efficiency can be enhanced. However, in the present disclosure, the acoustic coupling agent is not an essential component, and the ultrasonic wave irradiation apparatus need not use the acoustic coupling agent. In a case where the acoustic coupling agent B1 is not used in the present embodiment, since the sample plate 1 can be heated by friction at a contact portion between the ultrasonic wave generation device 2 and the sample plate 1 when the sample plate 1 is rotated relative to the ultrasonic wave generation device 2 by the rotation mechanism 4 to be described below, which is suitable for use in heating the target solution L1.

As illustrated in FIG. 1, the rear mass 23 is disposed below the drive unit 21 and is fixed to the piezoelectric elements 21a and 21b. That is, the rear mass 23 is disposed on the opposite side of the front mass 22 with the piezoelectric elements 21a and 21b interposed therebetween. The rear mass 23 is formed in a columnar shape extending along the up-down direction. A center axis of the rear mass 23 coincides with the rotation axis A1. That is, the rear mass 23 is disposed coaxially with the front mass 22. The rear mass 23 is formed with a through hole 23a extending along the up-down direction. The bolt 25 is inserted through the through hole 23a. The rear mass 23 functions as a counterweight for maintaining a weight balance with the front mass 22. In the present embodiment, the cross section of the rear mass 23 orthogonal to the rotation axis A1 is circular, and the shape is not limited thereto. For example, the cross section of the rear mass 23 may be polygonal.

The flange 24 is a member for supporting the ultrasonic wave generation device 2 on any structure. The flange 24 is formed in an annular shape, and the bolt 25 is inserted through the flange 24. The flange 24 is interposed between the drive unit 21 and the front mass 22 and protrudes radially outward. The flange 24 is disposed at a node of ultrasonic vibration generated by the ultrasonic wave generation device 2 (that is, a position where the amplitude of a wavelength is 0 and no vibration occurs). As illustrated in FIG. 1, in the present embodiment, the flange 24 is fixed to the housing 8 as a structure, so that the ultrasonic wave generation device 2 is supported by the housing 8. The material of the flange 24 is not particularly limited, and may be a metal material. In the present embodiment, the flange 24 is formed as a member separate from the front mass 22, but the flange 24 may be formed as a part of the front mass 22. That is, the front mass 22 and the flange 24 may be formed integrally.

The bolt 25 and the nut 26 are fastening members for fastening the drive unit 21, the front mass 22, the rear mass 23, and the flange 24. As illustrated in FIG. 1, the bolt 25 is formed as a stud bolt having no head portion, for example. The bolt 25 is inserted through the through hole 23a of the rear mass 23 along the up-down direction, and an upper end portion thereof reaches the front mass 22 via the drive unit 21 and the flange 24 and is screwed into the screw hole 22a. A lower end portion of the bolt 25 protrudes downward from the rear mass 23, and the nut 26 is screwed to the lower end portion of the bolt 25. By tightening the nut 26, the front mass 22, the drive unit 21, and the rear mass 23 are fixed together and integrated. Thus, the ultrasonic wave generation device 2 is configured as a bolt-fastened Langevin type transducer.

### Lid Member

The lid member 3 is a member that enables opening and closing of the open end 22e of the container installation part 22b formed as a recessed part. As illustrated in FIG. 1, the lid member 3 is formed in, for example, a plate shape (a disk shape in the present example), and is disposed at the upper end portion of the peripheral wall portion 222 in a direction orthogonal to the up-down direction. The open end 22e formed at the upper end portion of the peripheral wall portion 222 is closed by the lid member 3, so that the container installation part 22b is formed as a shielded space where light from the outside is blocked. The lid member 3 is provided with a shaft support hole 3a for supporting the shaft portion 42 of the rotation mechanism 4, and an irradiation hole 3b for enabling the light irradiation device 6 to irradiate the target solution L1 in the container installation part 22b with light. The shaft support hole 3a and the irradiation hole 3b are formed as through holes penetrating the lid member 3 in the up-down direction. A temperature control device 5 (to be described below) such as a heater can be installed on an inner side of the lid member 3, and the temperature of the target solution L1 in the well 13 can be controlled.

### Rotation Mechanism

The rotation mechanism 4 rotates the sample plate 1 installed in the container installation part 22b relative to the ultrasonic wave generation device 2. As illustrated in FIG. 1, the rotation mechanism 4 includes the shaft receiving hole 41, the shaft portion 42, and a rotation device 43.

The shaft receiving hole 41 is a through hole penetrating the ultrasonic wave generation device 2 along the rotation axis A1. Specifically, the shaft receiving hole 41 penetrates the front mass 22 and the bolt 25 in the up-down direction.

The shaft portion 42 is a shaft member extending along the up-down direction. The shaft portion 42 is inserted through the shaft receiving hole 41 and is connected to the sample plate 1 and thus it can rotate integrally with the sample plate 1. A center axis of the shaft portion 42 coincides with the rotation axis A1. An upper end portion of the shaft portion 42 penetrates the sample plate 1, the temperature control device 5, and the lid member 3 and protrudes upward from the lid member 3. A lower end portion of the shaft portion 42 penetrates the bolt 25, protrudes downward from the bolt 25, and is connected to the rotation device 43.

The rotation device 43 rotates the shaft portion 42 about the rotation axis A1. The rotation device 43 is disposed below the ultrasonic wave generation device 2. That is, the rotation device 43 is disposed on the opposite side of the container installation part 22b with respect to the piezoelectric elements 21a and 21b in the up-down direction. The shaft portion 42 protruding downward from the ultrasonic wave generation device 2 is connected to the rotation device 43. The rotation device 43 includes, for example, a motor as a drive source and a gear that transmits a rotational drive force of the motor to the shaft portion 42, and rotates the shaft portion 42 at an appropriate rotation speed. The configuration of the rotation device according to the present disclosure is not limited thereto, and any means can be adopted.

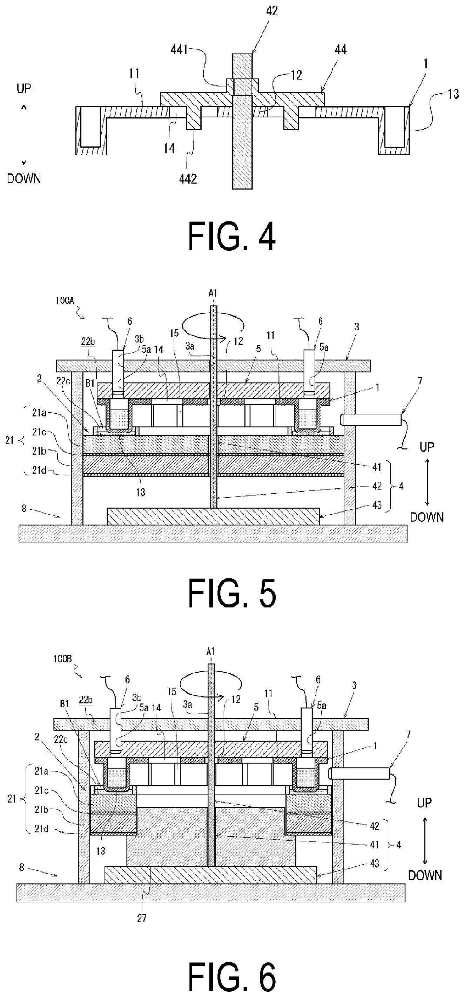

FIG. 4 is a diagram illustrating an example of a mode of connection between the shaft portion 42 of the rotation mechanism 4 and the sample plate 1. As illustrated in FIG. 4, the rotation mechanism 4 may include a coupling member 44 that couples the shaft portion 42 and the sample plate 1. The coupling member 44 includes a fixing portion 441 and an engaging portion 442. The fixing portion 441 is formed in a tubular shape, and is fitted to the shaft portion 42 and fixed to the shaft portion 42. The engaging portion 442 is inserted into the through hole 14 of the sample plate 1 to engage with the sample plate 1. Thus, when the shaft portion 42 rotates about the rotation axis A1, the sample plate 1 rotates integrally with the shaft portion 42.

### Temperature Control Device

The temperature control device 5 is disposed in the container installation part 22b, and adjusts (controls) the temperature of the target solution L1 contained in the sample plate 1. As illustrated in FIG. 1, the temperature control device 5 is formed in, for example, a disk shape (plate shape), and is installed in a manner that it overlaps the sample plate 1 from the upper side. The temperature control device 5 measures, for example, the temperature in the wells 13 of the sample plate 1, and controls the target solutions L1 contained in the wells 13 to a predetermined temperature, based on the measured temperature. The temperature control device 5 is provided with an irradiation hole 5a allowing the light irradiation device 6 to irradiate the target solution L1 in the container installation part 22b with light. The irradiation hole 5a is formed as a through hole penetrating the temperature control device 5 in the up-down direction. The configuration of the temperature control device according to the present disclosure is not limited thereto, and any means can be adopted. The temperature control device may include, for example, a heating device such as a heating wire heater or a heat pump, or a cooling device such as a heat exchanger or a Peltier element cooler.

### Light Irradiation Device

The light irradiation device 6 irradiates the target solution L1 with excitation light having a predetermined wavelength. The light irradiation device 6 includes, for example, a light-emitting diode as a light-emitting element (light source) that emits excitation light. However, the configuration of the light irradiation device 6 is not limited thereto. The light irradiation device 6 is inserted into the irradiation hole 3b of the lid member 3 and the irradiation hole 5a of the temperature control device 5, and emits excitation light into the well 13 from above the well 13. When viewed along the rotation axis A1, the light irradiation device 6 is disposed on the orbit of the plurality of wells 13. The light irradiation device 6 may be provided corresponding to each well 13. The light irradiation device 6 may emit excitation light through the window portion 22d formed in the front mass 22.

### Light Detection Device

The light detection device 7 detects fluorescence emitted from the fluorescent molecules when the excitation light is emitted by the light irradiation device 6. The light detection device 7 includes, for example, a phototransistor as a fluorescence receiving element that receives fluorescence. The light detection device 7 may include, for example, a photomultiplier tube as a fluorescence receiving element that receives fluorescence. However, the configuration of the light detection device 7 is not limited thereto. The light detection device 7 receives fluorescence in the well 13 from the side of the well 13 through the window portion 22d of the front mass 22.

The housing 8 accommodates the ultrasonic wave generation device 2. The material of the housing 8 is not particularly limited, and may be a metal material, for example. The housing 8 supports the flange 24 of the ultrasonic wave generation device 2.

### Operation

The operation of the ultrasonic wave irradiation apparatus 100 is described below by taking, as an example, detection of an aggregate of proteins using the ultrasonic wave irradiation apparatus 100 according to the present embodiment. The following describes, as an example, a case in which aggregates (amyloid fibrils) of proteins (β2 microglobulin) are generated in the target solution L1 including β2 microglobulin as an amyloidogenic protein, thioflavin T as a fluorescent molecule, sodium chloride (NaCl), and hydrochloride (HCl) and are detected by ThT fluorescence (thioflavin T fluorescence) by using the ultrasonic wave irradiation apparatus 100. The target solution L1 having, for example, a β2 microglobulin concentration of 0.3 mg/mL, a NaCl concentration of 150 mM, a HCl concentration of 30 mM, and a thioflavin T concentration of 5 µM is used. The resonance frequency of the ultrasonic wave generation device 2 configured as a Langevin type transducer is 23 kHz. However, the present disclosure is not limited thereto.

First, the acoustic coupling agent B1 is filled into the holding portion 22c formed in the container installation part 22b of the front mass 22, and the sample plate 1 in which the target solution L1 is contained in the plurality of wells 13 is installed in the container installation part 22b. At this time, the plurality of wells 13 are received by the holding portion 22c, so that parts including the bottom portions of the wells 13 are immersed in the acoustic coupling agent B1. Thus, the acoustic coupling agent B1 is interposed between the front mass 22 and the well 13 of the sample plate 1. The temperature control device 5 sets the measurement temperature to, for example, 37°C.

When the ultrasonic wave generation device 2 is operated in this state, ultrasonic waves are generated by the vibration of the piezoelectric elements 21a and 21b. The ultrasonic wave generation device 2 generates ultrasonic waves having a frequency of 23 kHz to 25 kHz, for example. The frequency of the ultrasonic waves may be a fixed value or may be swept within a designated range. The ultrasonic waves may be continuously generated, or generation and cessation thereof may be repeated in a designated cycle. The ultrasonic waves generated by the piezoelectric elements 21a and 21b are propagated through a solid by the front mass 22, and are emitted, via the acoustic coupling agent B1, to the plurality of wells 13 of the sample plate 1 installed in the container installation part 22b. Thus, the target solutions L1 contained in the plurality of wells 13 are irradiated with the ultrasonic waves. The ultrasonic waves are emitted to the target solutions L1 through the bottom portions of the wells 13. By irradiating the target solution L1 with the ultrasonic waves, the aggregation of proteins is accelerated and aggregates (amyloid fibrils) are generated.

At this time, the rotation mechanism 4 rotates the sample plate 1 about the rotation axis A1. That is, the irradiation of the target solution L1 with the ultrasonic waves is performed in a state where the sample plate 1 is rotated about the rotation axis A1 by the rotation mechanism 4 or in a state where the rotation and the stop are repeated. The rotation device 43 rotates the shaft portion 42 about the rotation axis A1, and thus the sample plate 1 connected to the shaft portion 42 rotates about the rotation axis A1. The shaft portion 42 is supported by the shaft support hole 3a of the lid member 3, so that whirling (shaft runout) of the shaft portion 42 is suppressed. The rotation mechanism 4 rotates the sample plate 1 at a rotation speed of 0.1 Hz, for example. Since the holding portion 22c is formed as an annular groove extending in the circumferential direction about the rotation axis A1, the plurality of wells 13 rotate (revolve) about the rotation axis A1 along the holding portion 22c. Thus, the target solutions L1 contained in the plurality of wells 13 are uniformly irradiated with ultrasonic waves. In addition, the sample plate 1 is rotated, and thus the target solutions L1 contained in the respective wells 13 are stirred.

Subsequently, in a state where the plurality of wells 13 are rotating, the light irradiation device 6 irradiates the solution in each well 13 with excitation light via the transparent sealing member. The light irradiation device 6 emits light having passed through a short-pass filter having a cutoff wavelength of 450 nm, for example, as the excitation light.

When the light irradiation device 6 irradiates the target solution L1 in the well 13 with excitation light, fluorescent molecules are bonded (adsorbed) to aggregates generated in the target solution L1, thereby emitting fluorescence. The light detection device 7 detects fluorescence emitted by the fluorescent molecules. As described above, since the sample plate 1 is a transparent body, the fluorescence from the target solution L1 in each well 13 passes through the sidewall of each well 13. Therefore, the light detection device 7 receives the fluorescence in the well 13 through the window portion 22d of the front mass 22. Since the peak fluorescence wavelength of the ThT fluorescence is about 482 nm, the light detection device 7 detects light having passed through a long-pass filter having a cutoff wavelength of 475 nm, for example.

By detecting the fluorescence from the target solution L1, the aggregates of the proteins can be detected.

### Actions and Effects

As described above, the ultrasonic wave irradiation apparatus 100 according to the present embodiment includes the ultrasonic wave generation device 2 including the piezoelectric elements 21a and 21b (vibration elements) that generate ultrasonic waves by vibrating. The ultrasonic wave generation device 2 includes the container installation part 22b in which the sample plate 1 for containing the target solution L1 as an object to be irradiated with ultrasonic waves can be installed, and propagates the ultrasonic waves generated by the piezoelectric elements 21a and 21b to the sample plate 1.

That is, the ultrasonic wave irradiation apparatus 100 according to the present embodiment is configured to irradiate the target solution L1 with ultrasonic waves by solid propagation. On the other hand, in the case of the device in the related art that irradiates an object with ultrasonic waves by fluid propagation, since a large-scale treatment tank for immersing a container of the object in a fluid such as water is necessary, the entire apparatus is increased in size. In addition, the generation of air bubbles might reduce the ultrasonic intensity, and a fluid degassing device for suppressing such a reduction is a cause of an increase in the size of the entire apparatus. In contrast, since the ultrasonic wave irradiation apparatus 100 according to the present embodiment irradiates the target solution L1 with ultrasonic waves by solid propagation, a large-scale treatment tank for immersing the sample plate 1 in a fluid, a fluid degassing device, or the like is not necessary. Thus, the present embodiment can implement a reduction in the size of the ultrasonic wave irradiation apparatus 100 and simplification of the structure thereof. Furthermore, since the attenuation of ultrasonic waves is smaller in the solid propagation than in the fluid propagation, the ultrasonic wave irradiation apparatus 100 according to the present embodiment can improve the energy efficiency of the introduction of the ultrasonic waves into an object and implement low output compared with the above-described device in the related art. In addition, since the ultrasonic wave irradiation apparatus 100 according to the present embodiment adopts a configuration in which an object is irradiated with ultrasonic waves by solid propagation, unevenness of ultrasonic waves can be reduced and reproducibility is excellent compared with the above-described device in the related art.

The ultrasonic wave generation device 2 according to the present embodiment includes the front mass 22 that propagates the ultrasonic waves generated by the piezoelectric elements 21a and 21b through a solid, and the front mass 22 includes the container installation part 22b and propagates the ultrasonic waves generated by the piezoelectric elements 21a and 21b to the sample plate 1.

That is, in the ultrasonic wave irradiation apparatus 100 according to the present embodiment, the container installation part 22b in which the sample plate 1 is installed is formed in the front mass 22 that propagates ultrasonic waves through a solid, and the target solution L1 is irradiated with the ultrasonic waves by the solid propagation via the front mass 22. According to this configuration, since the container installation part 22b is formed in the front mass 22, a container for installing the sample plate 1 need not be provided separately from the ultrasonic wave generation device 2. Thus, according to the present embodiment, further miniaturization of the ultrasonic wave irradiation apparatus 100 can be implemented. In addition, by providing the front mass 22 having high temperature resistance, ultrasonic irradiation under high temperature or low temperature becomes possible, and temperature adjustment becomes easy. As a result, the ultrasonic waves can be stably emitted even at a temperature higher than room temperature (for example, higher than 40°C). Furthermore, by enlarging the aperture of the front mass 22, the irradiation area of the ultrasonic waves can be increased. As a result, ultrasonic irradiation over a large area is possible. However, in the present disclosure, the first propagation part is not an essential component.

In addition, the container installation part 22b according to the present embodiment is provided with the holding portion 22c that holds, between the sample plate 1 and the holding portion 22c, the acoustic coupling agent B1 that can propagate ultrasonic waves.

According to this configuration, the use of the acoustic coupling agent B1 can suppress the attenuation of the ultrasonic waves generated by the ultrasonic wave generation device 2 when the ultrasonic waves are transmitted from the front mass 22 to the sample plate 1. As a result, the target solution L1 contained in the sample plate 1 can be efficiently irradiated with the ultrasonic waves. Note that in the present disclosure, the acoustic coupling agent is not an essential component.

The ultrasonic wave irradiation apparatus 100 according to the present embodiment further includes the rotation mechanism 4. The rotation mechanism 4 rotates the sample plate 1 installed in the container installation part 22b relative to the ultrasonic wave generation device 2 about the rotation axis A1 arranged along the up-down direction (the arrangement direction of the piezoelectric elements 21a and 21b and the container installation part 22b).

According to this configuration, by rotating the sample plate 1, the target solution L1 contained in the sample plate 1 can be uniformly irradiated with ultrasonic waves. This makes it possible to suppress variation in the influence of the ultrasonic waves (aggregation in the present example) on the sample plate 1. In addition, by rotating the sample plate 1, the target solution L1 can be stirred without providing a stirrer for stirring the target solution L1.

The rotation mechanism 4 according to the present embodiment includes the shaft receiving hole 41 penetrating the ultrasonic wave generation device 2 along the rotation axis A1, the shaft portion 42 inserted through the shaft receiving hole 41 and connected to the sample plate 1 to rotate integrally with the sample plate 1, and the rotation device 43 to which the shaft portion 42 is connected and which rotates the shaft portion 42 about the rotation axis A1. The rotation device 43 is disposed on the opposite side (lower side in the present example) of the container installation part 22b with respect to the piezoelectric elements 21a and 21b in the up-down direction.

That is, the rotation mechanism 4 employs a configuration in which the shaft portion 42 is inserted through the shaft receiving hole 41 penetrating the ultrasonic wave generation device 2, and thus the rotation device 43 is installed on the opposite side of the container installation part 22b. According to this configuration, since no structure is necessary to install the rotation device 43 on the container installation part 22b side, that is, above the container installation part 22b, the ultrasonic wave irradiation apparatus 100 can be decreased in size compared with when the rotation device 43 is installed above the container installation part 22b.

The sample plate 1 according to the present embodiment includes one or more wells 13 that are arrayed in the circumferential direction about the rotation axis A1 and in which the object is accommodated. The container installation part 22b is provided with the holding portion 22c that holds, between the well 13 and the front mass 22, the acoustic coupling agent B1 that can propagate ultrasonic waves, and the holding portion 22c is formed as an annular groove extending in the circumferential direction about the rotation axis A1.

According to this configuration, by forming the holding portion 22c as an annular groove, the one or more wells 13 can rotate (revolve) about the rotation axis A1 along the holding portion 22c while maintaining the contact state with the acoustic coupling agent B1. This makes it possible to efficiently irradiate the target solution L1 with ultrasonic waves while rotating the sample plate 1. In addition, by forming the holding portion 22c as an annular groove, the amount of the acoustic coupling agent B1 used can be minimized.

The container installation part 22b according to the present embodiment is formed as a recessed part that can accommodate the sample plate 1 and has the open end 22e on one side (the upper side in the present example). The ultrasonic wave irradiation apparatus 100 further includes the lid member 3 that enables opening and closing of the open end 22e of the recessed part.

According to this configuration, by closing the open end 22e with the lid member 3, the container installation part 22b can be formed as a shielded space where light from the outside is blocked. This can reduce the influence of external light. Note that the direction in which the open end of the container installation part according to the present disclosure is not particularly limited and need not be upward.

In addition, the container installation part 22b according to the present embodiment is provided with the window portion 22d for performing fluorescence measurement of the target solution L1 from the outside of the container installation part 22b.

According to this configuration, the fluorescence measurement of the target solution L1 can be performed from the outside of the container installation part 22b. The window portion 22d may be formed in the lid member 3. The window portion according to the present disclosure may be formed in at least one of the container installation part and the lid member.

The ultrasonic wave irradiation apparatus 100 according to the present embodiment further includes the temperature control device 5 that can adjust the temperature of the target solution L1.

According to this configuration, the temperature of the target solution L1 contained in the sample plate 1 can be controlled to an appropriate temperature.

The ultrasonic wave generation device 2 according to the present embodiment includes the rear mass 23 that is disposed on the opposite side of the front mass 22 with the piezoelectric elements 21a and 21b interposed therebetween and that propagates ultrasonic waves generated by the piezoelectric elements 21a and 21b through a solid.

According to this configuration, the rear mass 23 is disposed on the opposite side of the front mass 22 with the piezoelectric elements 21a and 21b (vibration elements) interposed therebetween, whereby the weight balance with the front mass 22 can be maintained. In addition, the ultrasonic wave generation device 2 includes the rear mass 23, and thus is suitable for generating ultrasonic waves having a low frequency of, for example, less than 100 kHz. However, the ultrasonic wave generation device according to the present disclosure need not include the rear mass.

### Variations

Ultrasonic wave irradiation apparatuses according to variations of the embodiment are described below. In the following description, differences from the ultrasonic wave irradiation apparatus 100 described with reference to FIGS. 1 to 4 are mainly described. The same components as those of the ultrasonic wave irradiation apparatus 100 are denoted by the same reference signs and detailed descriptions thereof are omitted.

### First Variation

FIG. 5 is a longitudinal cross-sectional view of an ultrasonic wave irradiation apparatus 100A according to a first variation of the embodiment. As illustrated in FIG. 5, the ultrasonic wave irradiation apparatus 100A according to the first variation is different from the above-described ultrasonic wave irradiation apparatus 100 in that the ultrasonic wave generation device 2 does not include the front mass 22 and the rear mass 23 and the container installation part 22b is formed over the drive unit 21 (the piezoelectric elements 21a and 21b).

In the first variation, as a space where the sample plate 1 can be installed, the container installation part 22b is formed over the upper surface (axial end surface) of the piezoelectric elements 21a and 21b (vibration elements) having an annular shape. That is, the sample plate 1 is placed over the piezoelectric elements 21a and 21b. In addition, the holding portion 22c for holding the acoustic coupling agent B1 between the sample plate 1 and the holding portion 22c is formed in an annular shape on the upper surface of the piezoelectric elements 21a and 21b, and the piezoelectric elements 21a and 21b are in contact with the sample plate 1 with the acoustic coupling agent B1 interposed therebetween. The acoustic coupling agent B1 need not be interposed between the piezoelectric elements 21a and 21b and the sample plate 1, and the piezoelectric elements 21a and 21b may be in direct contact with the sample plate 1. The holes of the piezoelectric elements 21a and 21b constitute the shaft receiving hole 41 penetrating the ultrasonic wave generation device 2, and the shaft portion 42 of the rotation mechanism 4 is inserted through the shaft receiving hole 41. The number of piezoelectric elements is not particularly limited, and may be, for example, 1 to 4.

In the ultrasonic wave irradiation apparatus 100A according to the first variation, ultrasonic waves generated by the piezoelectric elements 21a and 21b are emitted to the target solution L1 by solid propagation via the piezoelectric elements 21a and 21b and the sample plate 1. According to the first variation, since the ultrasonic waves generated by the piezoelectric elements 21a and 21b are emitted to an object without passing through a propagation member such as the front mass 22, attenuation of the ultrasonic waves can be suppressed. As a result, energy efficiency can be enhanced. In addition, since the ultrasonic wave irradiation apparatus 100A according to the first variation includes no propagation member such as the front mass 22, the entire apparatus can be further decreased in size. In addition, from the viewpoint of temperature, for example, when the temperature is equal to or lower than room temperature (for example, equal to or lower than 40°C), the ultrasonic waves can be stably emitted even when the propagation member such as the front mass 22 is not used and the piezoelectric elements 21a and 21b are brought into contact with the sample plate 1. Therefore, the ultrasonic wave irradiation apparatus 100A according to the first variation can be suitably used in a biological reaction generally performed at 40°C or lower or a polymer polymerization reaction performed only by ultrasonic waves at about 25°C.

### Second Variation

FIG. 6 is a longitudinal cross-sectional view of an ultrasonic wave irradiation apparatus 100B according to a second variation of the embodiment. As illustrated in FIG. 6, the ultrasonic wave irradiation apparatus 100B according to the second variation is different from the above-described ultrasonic wave irradiation apparatus 100 in that the container installation part 22b is formed over the piezoelectric elements 21a and 21b (vibration elements) as in the first variation.

The drive unit 21 (piezoelectric elements 21a and 21b) according to the second variation extends annularly along the plurality of wells 13 circumferentially arrayed. That is, the piezoelectric elements 21a and 21b are present only on the circumference on which the wells 13 are disposed. In the second variation, as a space where the sample plate 1 can be installed, the container installation part 22b is formed over the upper surface (axial end surface) of the piezoelectric elements 21a and 21b. In addition, as in the first variation, the holding portion 22c holding the acoustic coupling agent B1 between the sample plate 1 and the holding portion 22c is formed in an annular shape over the upper surface of the piezoelectric elements 21a and 21b. The piezoelectric elements 21a and 21b are in contact with the sample plate 1 with the acoustic coupling agent B1 interposed therebetween. The acoustic coupling agent B1 need not be interposed between the piezoelectric elements 21a and 21b and the sample plate 1, and the piezoelectric elements 21a and 21b may be in direct contact with the sample plate 1. The number of piezoelectric elements is not particularly limited, and may be, for example, 1 to 4. As illustrated in FIG. 6, regions of the piezoelectric elements 21a and 21b on an inner side of the holding portion 22c are hollow. The ultrasonic wave generation device 2 according to the second variation includes a support member 27 that supports the drive unit 21 on an inner side of the drive unit 21. The support member 27 is provided with the shaft receiving hole 41 penetrating the ultrasonic wave generation device 2, and the shaft portion 42 of the rotation mechanism 4 is inserted through the shaft receiving hole 41.

With the ultrasonic wave irradiation apparatus 100B according to the second variation, the same actions and effects as those of the first variation can be obtained. That is, since the ultrasonic wave irradiation apparatus 100B according to the second variation includes no propagation member such as the front mass 22, energy efficiency can be enhanced and the entire apparatus can be further decreased in size. In addition, in the second variation, since the piezoelectric elements 21a and 21b are present only on the circumference on which the wells 13 are disposed, the wells 13 can be more efficiently irradiated with ultrasonic waves. As a result, energy efficiency can be further improved.

The embodiments of the ultrasonic wave irradiation apparatuses according to the present disclosure have been described above, and each of the aspects disclosed in the present specification can be combined with any of the other features disclosed therein.

### Description of the Reference Numerals and Symbols

1 Sample plate (example of "irradiation target container")
2 Ultrasonic wave generation device
3 Lid member
4 Rotation mechanism
5 Temperature control device
6 Light irradiation device
7 Light detection device
8 Housing
13 Well
21a, 21b Piezoelectric element
22 Front mass (example of "first propagation part")
22b Container installation part
22c Holding portion
23 Rear mass (example of "second propagation part")
41 Shaft receiving hole
42 Shaft portion
43 Rotation device
100, 100A, 100B Ultrasonic wave irradiation apparatus

## Claims

1. An ultrasonic wave irradiation apparatus comprising:
an ultrasonic wave generation device including a vibration element configured to generate ultrasonic waves by vibrating, wherein
the ultrasonic wave generation device includes a container installation part in which an irradiation target container configured to accommodate an object to be irradiated with the ultrasonic waves is to be installed, and propagates the ultrasonic waves generated by the vibration element to the irradiation target container through a solid.

2. The ultrasonic wave irradiation apparatus according to claim 1, wherein
the ultrasonic wave generation device further includes a first propagation part configured to propagate the ultrasonic waves through a solid, and
the first propagation part includes the container installation part and propagates the ultrasonic waves to the irradiation target container.

3. The ultrasonic wave irradiation apparatus according to claim 1 or 2, wherein the container installation part is provided with a holding portion holding an acoustic coupling agent between the ultrasonic wave generation device and the irradiation target container, the acoustic coupling agent being configured to propagate the ultrasonic waves.

4. The ultrasonic wave irradiation apparatus according to claim 1 or 2, further comprising:
a rotation mechanism configured to rotate the irradiation target container installed in the container installation part, relative to the ultrasonic wave generation device, about a rotation axis arranged along an arrangement direction of the vibration element and the container installation part.

5. The ultrasonic wave irradiation apparatus according to claim 4, wherein
the rotation mechanism includes:
a shaft receiving hole penetrating the ultrasonic wave generation device along the rotation axis;
a shaft portion inserted through the shaft receiving hole and connected to the irradiation target container in a manner that the shaft portion is rotatable integrally with the irradiation target container; and
a rotation device disposed on an opposite side of the container installation part with respect to the vibration element part in the arrangement direction, the rotation device being connected to the shaft portion and configured to rotate the shaft portion about the rotation axis.

6. The ultrasonic wave irradiation apparatus according to claim 4, wherein
the irradiation target container includes one or more containing parts arrayed in a circumferential direction about the rotation axis and configured to accommodate the object,
the container installation part is provided with a holding portion holding an acoustic coupling agent between the ultrasonic wave generation device and the one or more containing parts, the acoustic coupling agent being configured to propagate the ultrasonic waves, and
the holding portion is formed as an annular groove extending in the circumferential direction about the rotation axis.

7. The ultrasonic wave irradiation apparatus according to claim 1 or 2, wherein
the container installation part is formed as a recessed part configured to accommodate the irradiation target container and provided with an open end on one side, and
the ultrasonic wave irradiation apparatus further comprises a lid member enabling opening and closing of the open end of the recessed part.

8. The ultrasonic wave irradiation apparatus according to claim 7, wherein a window portion is formed in at least one of the container installation part and the lid member, the window portion allowing fluorescence measurement of the object to be performed from an outside of the recessed part.

9. The ultrasonic wave irradiation apparatus according to claim 1 or 2, further comprising:
a temperature control device configured to adjust a temperature of the object.

10. The ultrasonic wave irradiation apparatus according to claim 2, wherein the ultrasonic wave generation device includes a second propagation part disposed on an opposite side of the first propagation part with the vibration element interposed between the second propagation part and the first propagation part, the second propagation part being configured to propagate the ultrasonic waves generated by the vibration element through a solid.

11. The ultrasonic wave irradiation apparatus according to claim 1, wherein the container installation part is formed over the vibration element.

12. The ultrasonic wave irradiation apparatus according to claim 11, wherein
the irradiation target container includes one or more containing parts circumferentially arrayed and configured to accommodate the object, and
the vibration element extends annularly along the one or more wells.

13. The ultrasonic wave irradiation apparatus according to claim 1 or 2, further comprising:
the irradiation target container.
